(19) Europäisches Patentamt
European Patent Office
Office européen des brevets

(11) Veröffentlichungsnummer: **0 011 784**
**B1**

# (12) EUROPÄISCHE PATENTSCHRIFT

(45) Veröffentlichungstag der Patentschrift: **24.03.82**

(51) Int. Cl.³: **C 07 D 229/00,** C 07 C 139/14, C 07 C 143/38 // C08G18/77

(21) Anmeldenummer: **79104540.4**

(22) Anmeldetag: **16.11.79**

(54) **Dispersionen aromatischer Isocyanatosulfonsäure-uretdione in organischen Polyisocyanaten und ein Verfahren zu ihrer Herstellung.**

(30) Priorität: **28.11.78 DE 2851341**

(43) Veröffentlichungstag der Anmeldung: **11.06.80 Patentblatt 80/12**

(45) Bekanntmachung des Hinweises auf die Patenterteilung: **24.03.82 Patentblatt 82/12**

(84) Benannte Vertragsstaaten: **BE DE FR GB IT NL**

(56) Entgegenhaltungen:
**EP-A-0 004 277**
**DE-A-2 227 111**
**DE-A-2 359 615**
**DE-A-2 640 103**

(73) Patentinhaber: **BAYER AG, Zentralbereich Patente, Marken und Lizenzen, D-5090 Leverkusen 1, Bayerwerk (DE)**

(72) Erfinder: **Dieterich, Dieter, Dr., Ludwig-Girtler-Strasse 1, D-5090 Leverkusen 1 (DE)**

**EP 0 011 784 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Dispersionen aromatischer Isocyanatosulfonsäure-uretdione in organischen Polyisocyanaten und ein Verfahren zu ihrer Herstellung

Bei der Sulfonierung aromatischer Isocyanate in flüssiger oder in organischen Lösungsmitteln gelöster Form werden feste Isocyanatosulfonsäure-uretdione erhalten, die in den meisten Fällen aus grobteilige Suspensionen anfallen (vgl. z. B. DE-OS 2 524 476.

Durch Filtrieren der Suspensionen lassen sich die Isocyanatosulfonsäure-uretdione in Pulverform gewinnen. Häufig werden für weitere Umsetzungen der Isocyanatosulfonsäuren, beispielsweise mit Oxiranen oder Oxetanen (vgl. z. B. DE-OS 2 651 065 oder deutsche Patentanmeldung P 27 35 047.6), die bei der Sulfonierung erhaltenen Suspensionen von Isocyanatosulfonsäure-uretdionen in unverändertem Ausgangsisocyanat oder auch in organischen Lösungsmitteln eingesetzt. In diesen Fällen macht die Handhabung dieser Suspensionen infolge ihrer Neigung zur Sedimentation Schwierigkeiten. Eine genaue Dosierung ist praktisch unmöglich. Ferner setzt der grobdisperse Charakter der Isocyanatosulfonsäuren ihre Reaktionsfähigkeit gegenüber Oxiranen bzw. Oxetanen herab, so dass beispielsweise bei der Herstellung von Schaumstoffen keine hinreichend rasche Auflösung der dispersen Phase erfolgt. Dies gilt insbesondere für Suspensionen der dimeren Sulfonsäure des Isocyanatotoluols bzw. des Diisocyanatodiphenylmethans in überschüssigem Toluylendiisocyanat bzw. Diisocyanatodiphenylmethan.

Es war daher die Aufgabe der vorliegenden Erfindung sedimentationsstabile Dispersionen von aromatischen Isocyanatosulfonsäure-uretdionen zur Verfügung zu stellen, die nicht mit den genannten Nachteilen behaftet sind.

Durch das nachstehend näher beschriebene erfindungsgemässe Verfahren gelang es nun erstmals, diese Aufgabe zu erfüllen.

Gegenstand der Erfindung sind feinteilige, sedimentationsstabile bei Raumtemperatur flüssige oder unter Erwärmen auf maximal 60°C verflüssigbare Dispersionen feinteiliger fester aromatischer Isocyanatosulfonsäure-uretdione einer unter 0,02 mm liegenden mittleren Teilchengrösse in organischen Polyisocyanaten, in denen, bezogen auf die Gesamtzahl der Äquivalente an Isocyanatgruppen insgesamt 5–60 Äquivalent-% an freien und dimerisierten Isocyanatgruppen von aromatischen Polyisocyanatosulfonsäuren vorliegen.

Gegenstand der vorliegenden Erfindung ist auch ein Verfahren zur Herstellung derartiger Dispersionen durch Sulfonierung von aromatischen Polyisocyanaten, dadurch gekennzeichnet, dass man entweder

a) aromatische Polyisocyanate mit 0,05 bis 0,6 Mol Schwefeltrioxid pro Mol Polyisocyanat oder einer entsprechenden Menge eines Schwefeltrioxid enthaltenden oder bildenden Sulfonierungsmittels in Gegenwart von 0,2 bis 25 Gew.-%, bezogen auf das Gewicht des Polyisocyanats einer hydrophoben, keine gegenüber Isocyanatgruppen inerte hydrophile Substituenten aufweisenden, in Isocyanaten zumindest bei erhöhter Temperatur löslichen, bei Raumtemperatur festen oder flüssigen, polare Gruppen aufweisenden, gegebenenfalls gegenüber Isocyanatgruppen reaktionsfähigen Gruppen aufweisenden, organischen Substanz sulfoniert oder

b) aromatische Polyisocyanate in Anwesenheit von 0,2 bis 25 Gew.-% bezogen auf das Gewicht des Polyisocyanats einer organischen Substanz der unter a) genannten Art in inerten, niedrig siedenden organischen Lösungsmitteln mit 0,2 bis 2,0 Mol Schwefeltrioxid pro Mol Polyisocyanat oder einer entsprechenden Menge eines Schwefeltrioxid enthaltenden oder bildenden Sulfonierungsmittels sulfoniert, anschliessend das Lösungsmittel destillativ entfernt, wobei bei Verwendung von mehr als 60 Mol-% Schwefeltrioxid pro Mol Polyisocyanat vor der destillativen Entfernung des Lösungsmittels das den wesentlichen Teil der kontinuierlichen Phase bildende Polyisocyanat zugesetzt wird, oder

c) instabile Grobsuspensionen von aromatischen Isocyanatosulfonsäure-uretdionen in Polyisocyanaten oder inerten Lösungsmitteln in Gegenwart von 0,2 bis 25 Gew.-%, bezogen auf die Gesamtisocyanatmenge, an unter a) definierten organischen Substanzen kurzzeitig bis zum Entstehen einer Lösung auf maximal 150°C erhitzt und rasch abkühlt und/oder einer Mahlung unterwirft, wobei gegebenenfalls mitverwendetes organisches Lösungsmittel destillativ entfernt wird und im Falle der Verwendung von Grobsuspensionen, welche keine nicht-sulfonierten Polyisocyanate enthalten das die kontinuierliche Phase bildende organische Polyisocyanat vor der destillativen Entfernung des Lösungsmittels hinzugegeben wird, und wobei jeweils die Menge an aromatischem Isocyanatosulfonsäure-uretdion und nicht sulfoniertem Polyisocyanat so bemessen wird, dass in den letztlich erhaltenen stabilen Dispersionen, bezogen auf die Gesamtmenge der Äquivalente an Isocyanatgruppen insgesamt 5 bis 60 Äquivalent-% an freien und dimerisierten Isocyanatgruppen des Isocyanatosulfonsäureuretdions vorliegen.

In der DE-OS 2 640 103 wird die Herstellung von Suspensionen von Isocyanatosulfonsäuren in bestimmten Suspendiermitteln beschrieben, wobei auf Seite 8 der Hinweis gegeben wird, dass der Zusatz von Tensiden im allgemeinen zu einer Verringerung der Teilchengrösse führt. Es war im Hinblick auf diese Lehre des Standes der Technik besonders überraschend, dass die Herstellung von sedimentationsstabilen Dispersionen von aromatischen Isocyanatosulfonsäure-uretdionen in organischen Polyisocyanaten als kontinuierlicher Phase unter Verwendung von hydrophoben, keinen Tensid-Charakter aufweisenden organischen Substanzen gelingt. Die genannte Vorveröffentlichung beschreibt im übrigen lediglich grobteilige Suspensionen von Isocyanatosulfonsäure-uretdionen in organischen Polyisocyanaten, die instabil sind und deren disperse Phase abgesaugt werden kann (Beispiel 1) oder grob- bzw. feinteilige Dispersionen in Hilfslösungsmitteln und beinhaltet keinerlei Hinweis, wie feinteilige Dispersionen der erfindungsgemässen Art in organischen Polyisocyanaten als kontinuierliche Phase zugänglich sein könnten.

Die DE-OS 2 227 111 betrifft lediglich flüssige Sulfonsäure- und/oder Sulfonatgruppe aufweisende aromatische Polyisocyanate, deren Viskosität durch Zusatz von flüssigen Diisocyanaten erniedrigt werden kann. Irgendwelche Hinweise auf feste Isocyanatosulfonsäure-uretdione, geschweige denn auf feinteilige, sedimentationsstabile Suspensionen von festen aromatischen Isocyanatosulfonsäure-uretdionen in organischen Polyisocyanaten sind dieser Veröffentlichung nicht zu entnehmen.

Die DE-OS 2 359 615 erwähnt beispielsweise im Beispiel 7 die Möglichkeit, bei höherer Temperatur flüssige, bei Raumtemperatur harzartige Sulfonierungsprodukte von flüssigen Polyisocyanatgemischen der Diphenylethanreihe mit N, N-Dibutylanilin zu neutralisieren, um so zu einem Sulfonatgruppen aufweisenden Produkt zu gelangen. Gleichzeitig wird hervorgehoben, dass die Viskosität bei dieser Neutralisation etwas abnimmt. Da allgemein bekannt ist, dass die Viskosität von Suspensionen bei konstantem Feststoffgehalt mit steigender Teilchengrösse abnimmt, konnte hieraus nicht hergeleitet werden, dass es möglich sein könnte, nach dem erfindungsgemässen Verfahren besonders feinteilige und sedimentationsstabile Suspensionen zu erhalten.

In den erfindungsgemässen Dispersionen liegen aromatische Isocyanatosulfonsäure-uretdione als disperse und beliebige flüssige Sulfonsäuregruppen-freie organische Polyisocyanate als kontinuierliche Phase vor, wobei sich die Gesamtzahl der Äquivalente an Isocyanatgruppen inklusive den in dimerisierter Form als Uretdion vorliegenden Isocyanatgruppen der Isocyanatosulfonsäure-uretdione zu 5 bis 60 Äquivalent-%, vorzugsweise 20 bis 52 Äquivalent-% aus Isocyanatgruppen bzw. dimerisierten Isocyanatgruppen der Isocyanatosulfonsäure-uretdione und 40 bis 95, vorzugsweise 48 bis 80 Äquivalent-% aus Isocyanatgruppen der nicht-sulfonierten Polyisocyanate zusammensetzen. Die die disperse Phase bildenden Isocyanatosulfonsäure-uretdione weisen im übrigen eine unter 0,02 mm, vorzugsweise unter 0,01 mm liegende mittlere Teilchengrösse auf, so dass sie von einem Sieb einer entsprechenden Maschenweite nicht zurückgehalten würden.

Die in den erfindungsgemässen Dispersionen als disperse Phase vorliegenden aromatischen Isocyanatosulfonsäure-uretdione stellen bei Raumtemperatur feste Verbindungen dar, wie sie bei der Sulfonierung von aromatischen Di- oder Polyisocyanaten in feinteiliger Form anfallen. Es handelt sich vorzugsweise um als Uretdionpolyisocyanate vorliegende Monosulfonierungsprodukte aromatischer Polyisocyanate der Formel

$$Q(NCO)_n$$

in welcher

Q für einen aromatischen Kohlenwasserstoffrest mit 6 bis 15 Kohlenstoffatomen und

n für eine ganze oder gebrochene Zahl von 2 bis 3 stehen.

Entsprechend der Definition von n als ganze oder gebrochene Zahl können selbstverständlich auch Polyisocyanatgemische einer mittleren, vorzugsweise zwischen 2 und 3 liegenden, NCO-Funktionalität eingesetzt werden. Geeignete Isocyanatosulfonsäure-uretdione sind jedoch auch Sulfonierungsprodukte von beliebigen substituierten, insbesondere Chlor, Brom-oder $C_1$-$C_4$-Alkoxy-substituierten aromatischen Polyisocyanaten oder die Sulfonierungsprodukte von Thioäther-, Carbodiimid-, Isocyanurat- oder Biuretgruppen aufweisenden aromatischen Polyisocyanaten. Beispiele geeigneter aromatischer Polyisocyanate sind 4,4'-Stilbendiisocyanat, 4,4'-Dibenzyldiisocyanat, 3,3'- bzw. 2,2'-Dimethyl-4,4'-diisocyanatodiphenylmethan, 2,5,2',5'-Tetramethyl-4,4'-diisocyanatodiphenylmethan, 3,3'-Dimethoxy-4,4'-diisocyanato-diphenylmethan, 3,3'-Dichlor-4,4'-diisocyanato-diphenylmethan, 4,4'-Diisocyanato-dimethylmethan, 4,4'-Diisocyanato-diphenylcyclohexylmethan, 4,4'-Diisocyanato-benzophenon, 4,4'-Diisocyanato-diphenylsulfon, 4,4'-Diisocyanato-diphenyläther, 4,4'-Diisocyanato-3,3'-dibrom-diphenylmethan, 4,4'-Diisocyanato-3,3'-diäthyl-diphenylmethan, 4,4'-Diisocyanato-diphenyl-äthylen-(1,2), 4,4'-Diisocyanato-diphenyl-sulfid, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4'-4''-triisocyanat, Polyphenyl-polymethylenpolyisocyanate, wie sie durch Anilin-Formaldehyd-Kondensation und anschliessend Phosgenierung erhalten und z. B. in den britischen Patentschriften 874 430 und 848 671 beschrieben werden, Carbodiimidgruppen aufweisende aromatische Polyisocyanate, wie sie in der deutschen Patentschrift 1 092 007 beschrieben werden, aromatische Diisocyanate, wie sie in der amerikanischen Patentschrift 3 492 330 beschrieben werden, Allophanatgruppen aufweisende aromatische Polyisocyanate, wie sie z. B. in der britischen Patentschrift 994 890, der belgischen Patentschrift 761 626 und der veröffentlichten holländischen Patentanmeldung 7 102 524 beschrieben werden, Isocyanuratgruppen aufweisende aromatische Polyisocyanate, wie sie z. B. in den deutschen Patentschriften 1 022 789, 1 22 067 und 1 027 394 sowie in den deutschen Offenlegungsschriften 1 929 034 und 2 004 048 beschrieben werden, acylierte Harnstoffgruppen aufweisende aromatische Polyisocyanate gemäss der deutschen Patentschrift 1 230 778, Biuretgruppen aufweisende aromatische Polyisocyanate, wie sie z. B. in der deutschen Patentschrift 1 101 394, in der britischen Patentschrift 889 050 und in der französischen Patentschrift 7 017 514 beschrieben werden.

Bevorzugt sind pulverförmige sulfonierte, in der dimeren Form vorliegende aromatische Di-und Triisocyanate, insbesondere die in Form der Dimeren vorliegenden Mono- und Disulfonsäuren vorzugsweise Monosulfonsäuren von 4,4'-Diisocyanato-diphenylmethan, 2,4'-Diisocyanato-diphenylmethan, und insbesondere 2,4-Diisocyanato-toluol und 2,6-Diisocyanato-toluol, sowie den aus diesen Isomeren bestehenden Gemischen.

Bei der kontinuierlichen Phase der erfindungsgemässen Dispersionen handelt es sich um beliebige organische Polyisocyanate, die jedoch vorzugsweise entweder bei Raumtemperatur flüssig oder durch einfaches Erwärmen auf maximal 60°C vorzugsweise maximal 40°C verflüssigbar sind. Die diesen Bedingungen entsprechenden obengenannten aromatischen Polyisocyanate stellen vorzugsweise die kontinuierliche Phase dar, daneben sind jedoch auch beispielsweise aliphatische Diisocyanate wie Hexamethylendiisocyanat, Trimethylhexamethylendiisocyanat oder cycloaliphatische Diisocyanate wie 1-Isocyanato-3,3,5-trimethyl-5-isocyanatomethylcyclohexan, 1-Methyl-2,4-diisocyanato-cyclohexan, 1-Methyl-2,6-diisocyanatocyclohexan, 4,4'-Diisocyanato-dicyclohexylmethan, sowie deren Dimere, Trimere, Allophanate und Biurete von erheblichem Interesse.

Bei der Durchführung des erfindungsgemässen Verfahrens ist die Mitverwendung von bestimmten Hilfsmitteln erforderlich. Bei diesen Hilfsmitteln handelt es sich um hydrophobe, keine gegenüber Isocyanatgruppen inerte hydrophile Substituenten aufweisenden, in den organischen Polyisocyanaten zumindest bei erhöhter Temperatur (maximal 150°C) lösliche, bei Raumtemperatur feste oder flüssige, polare Gruppen aufweisende, gegebenenfalls gegenüber Isocyanatgruppen reaktionsfähige Gruppen aufweisende, organische Substanzen. Zu diesen Substanzen gehören

1. den genannten Bedingungen entsprechende, überwiegend nicht-kristalline Polymere wie z. B. Polyvinylchlorid, Polyvinylidenchlorid, Polyvinylacetat, Polyacrylate, Polycarbonate, Celluloseester, Polypropylen, Polyester, Polyether, Polyurethane, Polystyrol, sowie Mischpolymerisate wie Styrol/Acrylnitril/Acrylat-Copolymere, Polyesterurethanharnstoffe, Polyvinylpyrrolidon. Die Molekulargewichte geeigneter Polymerer liegen im allgemeinen zwischen 4000 und 500 000, vorzugsweise zwischen 10 000 und 200 000. Besonders bevorzugt ist PVC.

2. Der obigen allgemeinen Definition entsprechende Verbindungen der Formel

R-X

in welcher

R für einen geradkettigen oder verzweigten aliphatischen, cycloaliphatischen oder aromatischen Kohlenwasserstoffrest mit 6 bis 30 Kohlenstoffatomen, dessen Wasserstoffatome ganz oder teilweise durch Halogenatome, insbesondere Fluor, Chlor oder Brom substituiert sein können, steht, und

X für -OH,
$-O-(CH_2-CH_2-O-)_n-H$,
-SH, -COOH, $-NH_2$, $-NHR^1$,
$-NR^1R^2$, $-CO-N\genfrac{}{}{0pt}{}{R^1}{R^2}$,
-NCO, $-(Y)_{0-1}-CO-NH-AR$,
$-(Y)_{0-1}-CO-NH-Ar-NCO$, $-NH-CO-Y-R_1$ steht, wobei

$R^1$ und $R^2$ für gleiche oder verschiedene $C_1$-$C_4$-Alkylreste stehen, welche durch -OH, $-O-(CH_2-CH_2O)_nH$, -SH, -Y-CO-NH-Ar oder -Y-CO-NH-Ar-NCO substituiert sein können,

Y für -O-, -S-, -NH oder $-NR^1-$ steht,

Ar für einen Rest steht wie er durch Entfernung einer Isocyanatgruppe aus einem Monoisocyanat des Molekulargewichtsbereichs 57 bis 180 oder wie er durch Entfernung einer oder zwei NCO-Gruppen aus einem organischen Polyisocyanat des Molekulargewichtsbereichs 168-300 erhalten wird und

n 1 oder 2 bedeutet.

Beispiele geeigneter Verbindungen der Gruppe 2 sind:

Alkohole bzw. Thioalkohole wie 1-Hexanol, 4-Methyl-2-pentanol, 2-Äthyl-1-butanol, 1-Octanol, 2-Äthyl-1-hexanol, 1-Nonanol, Trimethyl-1-hexanol, 1-Decanol, 1-Dodecanol, 1-Dodecanthiol, 1-Tetradecanol, 1-Hexadecanol, 1-Octadecanol, ferner technische Alkoholgemische wie «Lorol» ($C_{12}H_{26}O$-$C_{18}H_{38}O$), «Alfol» ($C_{14}H_{30}O$-$C_{22}H_{46}O$), Methyl-cyclohexanol, Cyclohexanmethanol, Trimethylcyclohexanol, Benzylalkohol, 2-Hydroxydecalin, 4-tert.-Butylcyclohexanol; Phenole wie Äthylphenol, Xylenol, Chlorxylenol, Isopropylphenol, sek.-Butylphenol, tert.-Butylphenol, Thymol, 4-(1,1-Dimethylpropyl)-phenol, Trichlorphenol, Trichlorkresol, 4-(1,1,3,3-Tetramethylbutyl)-phenol, Nonylphenol, Di-tert.-butyl-phenol, Di-tert.-pentyl-phenol, Dodecylphenol, Cyclohexylphenol, Naphthol, Phenyl-phenol, 4-Hydroxy-diphenyl, Benzylphenol, Cumylphenol sowie Isomerengemische und technische Gemische der aufgeführten Phenole.

Carbonsäuren wie 2-Äthylhexansäure, Kokosfettsäure-Vorlauf, Kokosfettsäure, Versaticsäure, Laurinsäure, Myristinsäure, Palmitinsäure, Stearinsäure, sowie technische Gemische derartiger Carbonsäuren, Tolylsäure, tert.-Butyl-benzoesäure, Naphthoesäure.

Amine wie 2-Äthyl-hexylamin, Bis-(2-äthylhexyl)-amin, Dodecylamin, Tetradecylamin, Hexadecylamin, Octadecylamin, Methyl-octadecylamin, N-Methylcyclohexylamin, N-Äthylcyclohexylamin, Dicyclohexylamin, Methylcyclohexylamin, Trimethylcyclohexylamin, N-Äthylanilin, N-Butylanilin, N-Isobutylanilin, Dioxäthyl-m-toluidin, Benzylanilin, O-Tolyl-benzylamin, Dibenzylamin, Xylidin, 3,5-Bis-trifluormethylanilin, Isopropylanilin, Äthylmethyl-anilin, Trimethylanilin, sek.-Butylanilin, tert.-Butylanilin, Diäthylanilin, Diäthylbutylanilin, Diisopropylanilin, Dodecylanilin, 4-Cyclohexylanilin, 1-Naphthylamin, N-Äthyl-1-naphthylamin, Cyclohexyl-amin; Isocyanate wie 6-Chlorhexylisocyanat, 2,6-Diisopropylphenylisocyanat, Dodecylisocyanat, Tetradecylisocyanat, Hexadecylisocyanat, Naphthylisocyanat, Stearylisocyanat.

Weitere Verbindungen der Gruppe 2 sind z. B. Kokosfettsäureamid, Laurinsäureamid, Stearylamid, Stearinsäuredimethylamid, Dodecyl-diäthanolamin, Oleyldiäthanolamin, Stearyl-diäthanolamin, Stearyl-diisopropanolamin, Dodecylsulfonsäure, Naphthalin-monosulfonsäure, Xylolsulfonsäure, Biphenyl-sulfonsäure, Pyrensulfonsäure, sulfonierte $C_8$-$C_{30}$-Kohlenwasserstoffe sowie Addukte aus den genannten Alkoholen, Aminoalkoholen, Phenolen, Mercaptanen, Carbonsäuren, Aminen und Amiden mit Mono-, Di- oder Polyisocyanaten.

3. Obiger allgemeiner Definition entsprechende, substituierte Kohlenwasserstoffe der Formel

R-Z-R′

in welcher

R die bereits angegebene Bedeutung hat,

R′ für einen gegebenenfalls Halogen- vorzugsweise Fluor-, Chlor- oder Brom-substituierten Kohlenwasserstoffrest mit 1 bis 18 Kohlenstoffatomen steht und

Z für eine -CO-, $-SO_2-$, -NHCONH-, -NHCOO-, -NHCO-, -NHCSNH-, -NH- oder -CHOH-Gruppe steht.

Beispiele geeigneter Verbindungen der Gruppe 3 sind Dicyclohexyl-keton, Ditolyl-keton, Acetophenon, p-Chloracetophenon, 2-Undecanon, 10-Nonadecanon, 1,1,3-Trimethyl-5-cyclohexanon, Cyclododecanon, Bis-dodecylharnstoff, Bisstearylharnstoff, N-Phenyl-N′-stearylharnstoff, N-Stearyläthylurethan, N-Methyl-stearyl-urethan, Distearylamid, Bis-stearyl(thio)harnstoff, Dicyclohexylamin, Dododecylamin, Distearylamin, 2-Undecanol, 10-Nonadecanol. Bevorzugt sind Addukte von Isocyanaten mit Alkoholen und Carbonsäuren.

4. Obiger allgemeiner Definition entsprechende, nicht unter die unter 2. genannte Formel fallende Addukte von organischen Monoisocyanaten, insbesondere aliphatischen Monoisocyanaten des Molekulargewichtsbereichs 155 bis 295 und Polyakoholen, Polyaminen oder Polycarbonsäuren, insbesondere aliphatischen Polyalkoholen des Molekulargewichtsbereichs 62 bis 300, bzw. nicht unter die unter 2. genannte Formel fallende Addukte von Polyisocyanaten des Molekulargewichtsbereichs 168–300 mit einwertigen Alkoholen, Monoaminen oder Monocarbonsäuren, insbesondere einwertigen aliphatischen Alkoholen des Molekulargewichtsbereichs 32–200, wobei die Addukte vorzugsweise mindestens eine aliphatische Kohlenwasserstoffkette mit 8 bis 30 Kohlenstoffatomen aufweisen sollten. Beispiele derartiger Addukte sind die Umsetzungsprodukte von

1 Mol TDI + 2 Mol Stearylalkohol
1 Mol TDI + 1 Mol Dodecanol + 1 Mol Methanol
1 Mol MDI + 1,8 Mol 2-Äthyl-hexanol
1 Mol BTI + 2 Mol Laurinsäure + 1 Mol Butanol
1 Mol HDI + 2 Mol Palmitinsäure
1 Mol TMP + 3 Mol Stearylisocyanat
1 Mol TMP + 2 Mol Stearylisocyanat
1 Mol GLY + 1 Mol Stearylisocyanat
1 Mol TMP + 1 Mol Stearylisocyanat + 2 Mol 6-Chlorhexylisocyanat
1 Mol HD + 1,5 Mol Laurylisocyanat
1 Mol AD + 2 Mol Stearylisocyanat
1 Mol WS + 2 Mol Stearylisocyanat

wobei die genannten Abkürzungen folgende Bedeutung haben:

TDI: 2,4-Toluylendiisocyanat,
MDI: 4,4′-Diisocyanatodiphenylmethan,
HDI: 1,6-Diisocyanatohexan,
BTI: Tris-(6-isocyanatohexyl)-biuret,
TMP: Trimethylolpropan
GLY: Glycerin
HD: Hexandiol (1,6)
AD: Adipinsäure
WS: Weinsäure

5. Andere unter die obige allgemeine Definition fallende organische Verbindungen wie z. B. Acetophenon, Diphenylsulfon, Adipinsäuredimethylester, Phthalsäuredioctylester oder Benzophenon.

Die unter 1.)–5.) beispielhaft genannten Hilfsmittel werden bei der Durchführung des erfindungsgemässen Verfahrens in Mengen von 0,2 - 25 Gew.-%, vorzugsweise 1-10 Gew.-% und insbesondere 2-6 Gew.-%, bezogen auf die Gesamtmenge der vorliegenden sulfonierten und nichtsulfonierten Polyisocyanate eingesetzt.

Die Durchführung des erfindungsgemässen Verfahrens zur Herstellung der erfindungsgemässen Dispersionen erfolgt vorzugsweise nach einer der folgenden Ausführungsformen a), b) oder c):

Ausführungsform a)

Ein aromatisches Polyisocyanat bzw. Polyisocyanatgemisch der oben beispielhaft genannten Art, welches vorzugsweise bei Raumtemperatur flüssig oder durch einfaches Erwärmen auf maximal 60 °C insbesondere maximal 40 °C verflüssigbar ist, wird in an sich bekannter Weise (vgl. hierzu z. B. US-PS 3 826 769, DE-OS 2 524 476 oder DE-OS 2 615 876) im Temperaturbereich zwischen -20 °C und 100 °C, vorzugsweise zwischen 0 °C und 60 °C und insbesondere zwischen 10 °C und 40 °C sulfoniert, wobei mindestens ein der unter 1.) bis 5.) beispielhaft genannten Hilfsmittel vor oder während der Sulfonierung zugesetzt wird. Während der Sulfonierung wird intensiv gerührt. Zur Sulfonierung wird hierbei Schwefeltrioxid oder ein beliebiges, Schwefeltrioxid enthaltendes oder abspaltendes Sulfonierungsmittel in einer solchen Menge verwendet, dass pro Mol an zu sulfonierendem aromatischem Polyisocyanat 0,05 bis 0,60 Mol, vorzugsweise 0,2 bis 0,52 Mol Schwefeltrioxid zur Einwirkung gelangen. Auf diese Weise entsteht direkt eine erfindungsgemässe Dispersion.

Ausführungsform b)

Ein aromatisches Polyisocyanat bzw. Polyisocyanatgemisch der oben beispielhaft genannten Art wird in an sich bekannter Weise beispielsweise gemäss DE-OS 2 615 876 in Anwesenheit eines erfindungswesentlichen Hilfsmittels der oben unter 1.) bis 5.) genannten Art in den angegebenen Mengen in einem inerten, niedrig siedenden organischen Lösungsmittel wie z. B. Methylenchlorid, Dichloräthan, Chloroform oder Tetrachloräthan im bereits unter a) genannten Temperaturbereich sulfoniert, wobei pro Mol an zu sulfonierendem aromatischen Polyisocyanat 0,2 bis 2,0, vorzugsweise 0,3 bis 1,2 Mol Schwefeltrioxid zur Anwendung gelangen können. Anschliessend wird das Lösungsmittel destillativ entfernt, wobei im Falle der Verwendung von mehr als 0,6 Mol Schwefeltrioxid pro Mol Polyisocyanat vor der destillativen Entfernung des Lösungsmittels ein, den wesentlichen Anteil der kontinuierlichen Phase bildendes organisches Polyisocyanat in einer solchen Menge zugesetzt wird, das letztlich der Gehalt der erhaltenen Dispersion an aromatischen Isocyanatosulfonsäure-uretdion den oben gemachten Angaben entspricht. Das die kontinuierliche Phase bildende Polyisocyanat sollte zweckmässigerweise entweder bei Raumtemperatur flüssig oder durch einfaches Erwärmen auf maximal 60°C verflüssigbar sein. Das zur Sulfonierung eingesetzte aromatische Polyisocyanat kann bei dieser Ausführungsform selbstverständlich einen über 60°C liegenden Schmelzpunkt aufweisen.

Bei beiden Ausführungsformen a) und b) werden sowohl zur Sulfonierung als auch als kontinuierliche Phase vorzugsweise die oben beispielhaft genannten bevorzugten aromatischen Polyisocyanate eingesetzt.

Ausführungsform c)

Gemäss dieser Ausführungsform des erfindungsgemässen Verfahrens werden Grobsuspensionen von aromatischen Isocyanatosulfonsäure-uretdionen in aromatischen Polyisocyanaten oder den bereits beispielhaft genannten leicht flüchtigen, inerten Lösungsmitteln eingesetzt. Man erhält derartige Grobsuspensionen, wenn man die Sulfonierung von aromatischen Polyisocyanaten gemäss Stand der Technik vornimmt (vgl. hierzu z. B. US-PS 3 826 769, DE-OS 2 524 476 oder DE-OS 2 615 876) oder auch, wenn man gemäss Stand der Technik, gewonnene pulverförmige aromatische Isocyanatosulfonsäureuretdione mit flüssigen Polyisocyanaten und/oder inerten Lösungsmitteln anschlämmt. Nach Zugabe der erfindungswesentlichen unter 1.)–5.) beispielhaft genannten Hilfsmittel in den angegebenen Mengenverhältnissen zu diesen Grobsuspensionen werden diese während einer möglichst kurzen Zeitspanne bis zum Eintritt der Lösung auf maximal 150°C erhitzt und anschliessend rasch abgekühlt, oder einer Mahlung unterworfen, wobei nach der Mahlung bzw. während der Hitzebehandlung das gegebenenfalls mitverwendete Hilfslösungsmittel destillativ entfernt wird, und wobei im Falle der Abwesenheit von nicht-sulfonierten Polyisocyanaten vor der destillativen Entfernung des Lösungsmittels ein flüssiges, die kontinuierliche Phase bildendes Polyisocyanat der oben bereits beschriebenen Art hinzugegeben wird.

Es ist zur Vermeidung nicht näher bekannter Nebenreaktionen günstig, wenn die Dauer des Erwärmungsvorgangs möglichst kurz ist, z. B. 2 Minuten bis ca. 2 Stunden, vorzugsweise 2 Minuten bis ca. 30 Minuten. Aus diesem Grund sollte das Aufheizen möglichst rasch erfolgen. Auch sollte die Temperatur nicht höher steigen, als zur Erzielung einer vorübergehenden Lösung unbedingt erforderlich ist. Eine Maximaltemperatur von 150°C sollte keinesfalls überschritten werden. Vorzugsweise liegt die zur Lösung kurzzeitig erforderliche Höchsttemperatur zwischen 80 und 120°C. Während des Aufheizens und insbesondere während des Abkühlens sollte gerührt bzw. mechanisch bewegt werden.

Bei kontinuierlicher Arbeitsweise ist es günstig, Wärmeaustauscher in einer Erhitzungszone mit kurzer Verweildauer anzuwenden.

Die bereits erwähnte Mahlung der Grobsuspensionen in Gegenwart der erfindungswesentlichen Hilfsmittel kann zusätzlich oder auch anstelle der Temperaturerhöhung vorgenommen werden. Die Massnahme dient zur Verkleinerung der Teilchengrösse der suspendierten aromatischen Isocyanatosulfonsäure-uretdione auf unter 0,02 mm und ist insbesondere bei hohen Konzentrationen an festem Isocyanatoarylsulfonsäureuretdion, bei denen unterhalb 150°C keine Lösung durch Erwärmen erzielt werden kann, erforderlich. Ferner empfiehlt es sich in Gegenwart von aromatischen Dispersionsmedien wie Toluol, Chlorbenzol, TDI oder MDI.

Die Feindispergierung durch Erhitzen und Wiederabkühlen findet vorzugsweise in Gegenwart aliphatischer Dispersionsmedien, wie insbesondere in Gegenwart von aliphatischen Polyisocyanaten Anwendung.

Bei der Ausführungsform c) des erfindungsgemässen Verfahrens ist die Verwendung von Polymeren insbesondere von PVC-Pulver als erfindungsgemäss einzusetzendes Hilfsmittel vorteilhaft, während die Verwendung dieser Hilfsmittel bei der Ausführungsform b) des erfindungsgemässen Verfahrens nur dann zweckmässig ist, wenn das Sulfonierungsmittel im Unterschuss gegenüber dem zu sulfonierenden Polyisocyanat eingesetzt wird, da anderenfalls Dunkelfärbung eintritt.

Die Ausführungsformen b) und c) des erfindungsgemässen Verfahrens unter nachträglicher Zugabe des die kontinuierliche Phase bildenden Polyisocyanats sind besonders gut zur Herstellung von Dispersionen in aliphatischen Polyisocyanaten geeignet. Im Hinblick auf die einwandfreie Toxikologie dieser aliphatischen Polyisocyanate, sowie ihre gute Handhabbarkeit durch flüssigen Aggregatzustand beanspruchen derartige Dispersionen ein besonderes Interesse.

Bei Verwendung von gegenüber Isocyanatgruppen reaktionsfähige Gruppen aufweisenden erfindungswesentlichen Hilfsmitteln ist es zweckmässig, wenn auch nicht erforderlich, diese mit dem Polyisocyanat vor der Durchführung des erfindungsgemässen Verfahrensmassnahmen insbesondere vor der Durchführung der Sulfonierungsreaktion gemäss Ausführungsformen a) und b) abreagieren zu lassen. Die sich aus diesen Hilfsmitteln, beispielsweise den beispielhaft genannten Alkoholen, Aminen oder Carbonsäuren bildenden Urethan-, Harnstoff- bzw. Amidisocyanate sind besonders wirksame Hilfsmittel.

Die beschriebenen Verfahrensvarianten a), b) und c) lassen sich selbstverständlich auch kombinieren. So kann man beispielsweise TDI in Gegenwart von Stearylisocyanat sulfonieren, die feinteilige Suspension anschliessend mit Polyvinylchloridpulver und/oder einem Fettalkoholgemisch kurz erhitzen (wobei keine Lösung einzutreten braucht, da die Suspension bereits feinteilig ist) und unter Rühren wieder abkühlen. Da auf diese Weise weder das PVC noch das Fettalkoholgemisch unmittelbar mit dem Sulfonierungsmittel in Berührung kommt, wird ein besonders helles Produkt erhalten.

Entsprechend erhält man eine besonders hellfarbene Suspension aromatischer Isocyanato-Sulfonsäureuretdione in aliphatischen Polyisocyanaten, wenn die Sulfonierung des aromatischen Isocyanats in Gegenwart von Stearylisocyanat und/oder einem aus aliphatischen Isocyanat und Fettalkoholgemisch oder Benzylalkohol hergestellten Urethan vorgenommen wird, anschliessend das 1-5% PVC enthaltende aliphatische Isocyanat zugesetzt und anschliessend gegebenenfalls das mitverwendete Lösungsmittel abdestilliert wird.

Durch den Zusatz der erfindungswesentlichen unter 1. bis 5. beispielhaft genannten Hilfsmittel, welche keinen Tensidcharakter aufweisen, wird die Teilchengrösse der anfallenden Isocyanatosulfonsäuren stark erniedrigt, so dass sedimentationsstabile Dispersionen erhalten werden können. Insbesondere ist es nach dem erfindungsgemässen Verfahren möglich, feinteilige sedimentationsstabile Dispersionen der dimeren Diisocyanatotoluolsulfonsäure im Diisocyanatotoluol, sowie der dimeren Diisocyanatodiphenylmethansulfonsäure in Diisocyanatodiphenylmethan herzustellen. Dies ist für die Handhabung solcher Dispersionen von grosser Bedeutung. Die Sulfonsäuren sind in den Dispersionen homogen verteilt, diese lassen sich exakt dosieren und ermöglichen infolge der grossen Teilchenoberfläche rasche Reaktion, beispielsweise mit cyclischen Äthern an der Sulfonsäuregruppe sowie mit Verbindungen mit aktiven Wasserstoffatomen an der Isocyanatgruppe. Wie bereits dargelegt, weisen die dispergierten Isocyanatoarylsulfonsäure-uretdione durchschnittliche Teilchendurchmesser von weniger als 0,02 mm und vorzugsweise von unter 0,01 mm auf. Häufig werden Durchmesser zwischen 0,0004 und 0,008 mm erhalten. Demgegenüber liegen die Durchmesser der Teilchen von gemäss Stand der Technik erhaltenen Grobsuspensionen zwischen 0,05 und 0,5 mm.

Die erfindungsgemässen Dispersionen sind sedimentationsstabil, das heisst, dass innerhalb 24 Stunden keine nennenswerte Sedimentation eintritt, während Dispersionen gemäss Stand der Technik bereits nach 0,5 Stunden deutlich sedimentieren. Wie in den Ausführungsbeispielen gezeigt werden wird, werden in vielen Fällen Dispersionen erhalten, die selbst nach 6 Monaten nur geringfügig sedimentieren.

Zu den bevorzugten erfindungsgemässen Dispersionen gehören solche von dimerisierter Diisocyanato-toluolsulfonsäure in Diisocyanatotoluol bzw. Diisocyanatodiphenylmethan, von dimerem, monosulfoniertem Diisocyanatodiphenylmethan in Diisocyanatodiphenylmethan, sowie Dispersionen der genannten aromatischen Isocyanatosulfonsäure-uretdione in den beispielhaft genannten flüssigen aliphatischen bzw. cycloaliphatischen Diisocyanaten.

Im Rahmen der vorliegenden Erfindung sind unter der Bezeichnung «Diisocyanatoarylsulfonsäure-uretdion» die Produkte zu verstehen, wie sie bei der Sulfonierung von überwiegend reinen aromatischen Polyisocyanaten oder ihren Isomerengemischen in fester kristalliner Form anfallen. Sie können anteilig auch Sulfonsäureanhydride enthalten. Unter der Bezeichnung Diisocyanatodiphenylmethan (als Dispersionsmedium) werden nicht nur die mehr oder weniger reinen Zweikern-Produkte, sondern auch die sogenannten MDI-Polymer-Typen also alle Phosgenierungsprodukte der Anilin-Formaldehyd-Kondensation verstanden; ebenso entsprechend aufgebaute Produkte, die z. B. durch Spaltung von Produkten der Phenylurethan-Formaldehyd-Kondensation oder nach dem Verfahren der DE-OS 2 709 490 erhalten wurden.

Die erfindungsgemässen Dispersionen können als neuartige Ausgangsmaterialien bei der Herstellung von Isocyanat-Polyadditionsprodukten und insbesondere bei der Herstellung von Polyurethanen in kompakter und geschäumter Form eingesetzt werden.

Sie ermöglichen den Einsatz fester sulfonierter Isocyanate mit hohem Sulfonierungsgrad in der für den Verarbeiter praktikablen Form flüssiger Produkte. Hieraus hergestellte Polyurethane bzw. Polyurethanharnstoffe zeigen ein stark verbessertes Brandverhalten. Bei der Umsetzung mit Oxiranen, Oxetanen, Tetrahydrofuran und Caprolacton werden durch die grosse Oberfläche der dispergierten Sulfonsäure hohe Reaktionsgeschwindigkeiten erzielt, wie sie zur Herstellung von Schaumstoffen erforderlich sind.

Dispersionen aromatischer Isocyanat-Sulfonsäure-Uretdione in aliphatischen Polyisocyanaten niedrigen Dampfdrucks sind Isocyanatsysteme, die untoxisch und in jeder Hinsicht umweltfreundlich sind.

In den nachfolgenden Beispielen beziehen sich alle Prozentangaben, soweit nicht anders lautend vermerkt, auf Gewichtsprozente.

Beispiel 1

522 g (3,0 Mol) «TDI 80» (Isomerengemisch aus 80% 2,4-Diisocyanatotoluol und 20% 2,6-Diisocyanatotoluol) werden mit 26,2 g (0,1 Mol) eines technischen Fettalkoholgemischs (2% $C_{14}H_{29}OH$, 51% $C_{16}H_{33}OH$, 28% $C_{18}H_{37}OH$, 14% $C_{20}H_{41}OH$, 3% $C_{22}H_{45}OH$; Schmp. 43-47°, Kp. 318°) 4 Stunden bei 50° gerührt. Unter Rühren werden bei 25-30°C 115 g (1,44 Mol) Schwefeltrioxid aufgeleitet, wobei eine sehr feinteilige makroskopisch homogene Dispersion des Sulfonsäureuretdions in überschüssigem TDI 80 in Form einer streichbaren Paste erhalten wird. Innerhalb von 6 Monaten tritt keinerlei Sedimentation ein. Gehalt an Sulfonsäure-Uretdion: 365 g = 55%; Gehalt an freien bzw. dimerisierten Isocyanatgruppen des sulfonierten Diisocyanats, bezogen auf Gesamtmenge aller Isocyanatgruppen: 48 Äquivalent-%.

Vergleichsbeispiel

Wird derselbe Versuch in Abwesenheit des Fettalkohols durchgeführt, so wird eine grobteilige Suspension des Sulfonsäure-uretdions erhalten, die innerhalb weniger Stunden sedimentiert.

Beispiel 2

522 g (3,0 Mol) TDI 80 werden mit 7,8 g (1,5%) handelsüblichem Polyvinylchlorid-Pulver bei 110°C verrührt, bis sich das PVC gelöst hat. Unmittelbar nach Abkühlen auf 22° werden unter Rühren 73 g (0,9 Mol) Schwefeltrioxid aufgeleitet, wobei eine sehr feinteilige Dispersion des Sulfonsäureuretdions in überschüssigem TDI 80 entsteht. Nach 6 Monaten ist die Dispersion eingedickt, jedoch nicht sedimentiert. Beim Rühren wird schwache Dilatanz beobachtet. Gehalt an Sulfonsäure-Uretdion 230 g = 38%; Gehalt an freien bzw. dimerisierten Isocyanatgruppen des sulfonierten Diisocyanats, bezogen auf Gesamtmenge aller Isocyanatgruppen: 30 Äquivalent-%.

Beispiel 3

522 g (3,0 Mol) TDI 80 werden mit 22,8 g (0,1 Mol) eines technischen Fettalkoholgemischs aus $C_{12}H_{25}OH$, $C_{14}H_{29}OH$, $C_{16}H_{33}OH$ und $C_{18}H_{37}OH$ 30 Minuten bei 22-27°C verrührt und anschliessend 114 g (1,43 Mol) Schwefeltrioxid aufgeleitet. Es wird eine überwiegend sehr feinteilige makroskopisch homogene Dispersion in Form einer streichfähigen Paste erhalten. Gehalt an Sulfonsäure-Uretdion: 362 g = 55%; Gehalt an freien bzw. dimerisierten Isocyanatgruppen des sulfonierten Diisocyanats, bezogen auf Gesamtmenge aller Isocyanatgruppen: 48 Äquivalent-%.

Beispiel 4

Auf das gerührte Gemisch aus 522 g (3,0 Mol) TDI 80 und 29,5 g (0,1 Mol) Stearylisocyanat werden bei 20-30° 64 g (0,8 Mol) Schwefeltrioxid aufgeleitet. Es wird eine sehr feinteilige thixotrope Dispersion in Form einer Paste erhalten. Nach 7 Monaten ist keine Sedimentation eingetreten; Gehalt an freien bzw. dimerisierten Isocyanatgruppen des sulfonierten Diisocyanats, bezogen auf Gesamtmenge aller Isocyanatgruppen: 27 Äquivalent-%.

Beispiel 5

12,5 g handelsübliches Polyvinylchlorid-Pulver wurden in 500 ml 1,2-Dichloräthan gelöst. Anschliessend werden 250 g (1,0 Mol) 4,4'-Diisocyanatodiphenylmethan und 14,7 g (0,05 Mol) Stearylisocyanat zugegeben und ebenfalls gelöst. Bei 26-30° werden unter Rühren 80 g (1 Mol) Schwefeltrioxid aufgeleitet. Es wird eine sehr feinteilige dunkle Dispersion erhalten. Nach 6 Monaten ist nur geringe Sedimentation eingetreten. Durch Rühren lässt sich rasch völlige Homogenisierung erreichen. Gehalt an Sulfonsäure-Uretdion: 33,6%. 100 g der Dispersion in Dichloräthan werden mit 80 g Hexamethylendiisocyanat vermischt und hierauf das Dichloräthan bei 50°C unter vermindertem Druck abdestilliert. Es wird eine feinteilige Dispersion des Isocyanatosulfonsäure-uretdions in Hexamethylendiisocyanat mit einem Feststoffgehalt von 29% erhalten.

Beispiel 6

Zu 261 g (1,5 Mol) TDI 80 werden 5,3 g N-Oleyldiäthanolamin zugetropft, wobei unter Temperaturanstieg Urethanisierung erfolgt. Anschliessend wird mit 39 g (0,49 Mol) Schwefeltrioxid sulfoniert. Es wird eine sehr feinteilige Dispersion erhalten, die innerhalb von 6 Monaten nur wenig sedimentiert und sehr leicht redispergierbar ist. Gehalt an Sulfonsäure-Uretdion: 124 g = 40,5%; Gehalt an freien bzw. dimerisierten Isocyanatgruppen des sulfonierten Diisocyanats, bezogen auf Gesamtmenge aller Isocyanatgruppen: 33 Äquivalent-%.

Beispiel 7

Es wird wie in Beispiel 2 gearbeitet jedoch unter Verwendung von 4 g eines hochmolekularen handelsüblichen Polycarbonats anstelle von PVC. Die Dispersion wird durch ein feines Metallsieb gegeben und so von grobteiligen Anteilen befreit. Das Filtrat ist eine feinteilige Dispersion.

Beispiel 8

Es wird wie in Beispiel 2 gearbeitet jedoch unter Verwendung von 4 g eines handelsüblichen hochmolekularen Polystyrols. Es wird eine dünnflüssige feinteilige Dispersion erhalten, die nur langsam sedimentiert. Der abgesetzte Niederschlag ist sehr leicht redispergierbar.

Beispiel 9 bis 23

In einer orientierenden Versuchsreihe wurden eine Reihe von Substanzen auf ihre emulgierende bzw. stabilisierende Wirkung bei der Sulfonierung von Toluylendiisocyanat getestet. Hierzu wurden 20 g TDI 80 mit 2 g der zu prüfenden Substanz vermischt und im Reagenzglas mit 5 ml einer 26 proz.-Lösung von Schwefeltrioxid in Dichloräthan vermischt. Da hierbei ohne Rühren praktisch momentan sulfoniert wird, fallen die Niederschläge verhältnismässig grobteilig aus und sedimentieren rasch. Auch mit Stearylisocyanat wird unter diesen Bedingungen ein Sediment erhalten, das allerdings feinteilig ist und sehr leicht redispergiert werden kann. Gute Ergebnisse, das heisst feinteilige langsam sedimentierende und leicht redispergierbare Niederschläge werden erhalten mit:

9. Dodecanol
10. Stearylisocyanat
11. Nonylphenol
12. Dicyclohexylamin
13. Kokosfettsäure
14. Stearinsäure
15. Naphthoesäure
16. Cyclohexylphenol
17. tert.-Butylphenol
18. Trichlorphenol
19. 2-Äthyl-1-hexanol
20. 1-Hexanol
21. Acetophenon
22. Benzylalkohol
23. Stearylisocyanat + Benzylalkohol (Niederschlag sedimentiert nicht)

Mit diesen Substanzen können unter den Bedingungen des Beispiels 1 sehr feinteilige nicht oder nur sehr langsam sedimentierende Dispersionen erhalten werden.

Beispiel 24

Eine Lösung von 0,3 g PVC-Pulver in 20 g TDI 80 wird mit 2 g Benzylalkohol versetzt und 1 Stunde unter Urethanisierung reagieren gelassen. Nach Zugabe von 10 ml einer 26-proz. Lösung von Schwefeltrioxid in Dichloräthan wird eine sehr feinteilige Dispersion erhalten, die sich innerhalb von 48 Stunden nicht absetzt.

Beispiel 25

100 g 1,6-Diisocyanatohexan und 5 g PVC-Pulver werden unter Rühren erhitzt bis das PVC vollständig in Lösung gegangen ist. Bei 100–130° werden 20 g des Uretdions der 2,4-Diisocyanatotoluol-5-sulfonsäure eingetragen und unter Rühren gelöst. Nach Abkühlen der Lösung erhält man eine sehr feinteilige sedimentationsstabile Dispersion.

Beispiel 26

174 g (1,0 Mol) TDI 65, 15 g Stearylisocyanat und 5 g Benzylalkohol werden in 250 ml 1,2-Dichloräthan gelöst. Bei 10–20° werden 80 g (1 Mol) Schwefeltrioxid aufgeleitet. Es wird eine sehr feinteilige weisse Dispersion erhalten. Anschliessend werden 350 g einer 5proz. Lösung von PVC in 1,6-Diisocyanatohexan eingerührt und Dichloräthan im Vaakum abgezogen. Es wird eine weisse, sehr feinteilige Dispersion erhalten, die völlig sedimentationsstabil ist. Gehalt an Uretdion-Sulfonsäure: 39%

Beispiel 27

Es wird wie in Beispiel 26 verfahren, jedoch unter Verwendung von Isophoron-diisocyanat anstelle von 1,6-Diisocyanatohexan. Die erhaltene weisse Isocyanat-Dispersion ist sehr gut handhabbar, verursacht bei Raumtemperatur keine Schleimhautreizungen durch Dämpfe und liefert beim hydrolytischen Abbau der daraus hergestellten Kunststoffe keine aromatischen Diamine.

Beispiel 28

Analog Beispiel 24 wird TDI 80 unter Zusatz von 15% 6-Chlorhexylisocyanat sulfoniert. Es wird eine feinteilige Dispersion erhalten, die sich innerhalb 24 Stunden nicht absetzt.

Beispiel 29

20 g des Uretdions der 2,4-Diisocyanatotoluol-5-sulfonsäure werden in 100 g Isophorondiisocyanat, welches 1% PVC enthält, gelöst auf 140° erhitzt, wobei eine klare Lösung entsteht und diese unter Rühren rasch auf Raumtemperatur wieder abkühlt. Es entsteht eine sehr feine opakweisse Dispersion, welche nicht sedimentiert. Ein entsprechendes Produkt wird erhalten, wenn anstelle von PVC 2% «Alfol 1620» (vgl. Beispiel 1) eingesetzt wird. Die Dispersion ist über Monate lagerstabil.

## Patentansprüche

1. Feinteilige, sedimentationsstabile, bei Raumtemperatur flüssige oder unter Erwärmen auf maximal 60°C verflüssigbare Dispersionen feinteiliger fester aromatischer Isocyanatosulfonsäure-uretdione einer unter 0,02 mm liegenden mittleren Teilchengrösse in organischen Polyisocyanaten, in denen, bezogen auf die Gesamtzahl der Äquivalente an Isocyanatgruppen insgesamt 5–60 Äquivalent-% an freien und dimerisierten Isocyanatgruppen von aromatischen Polyisocyanatosulfonsäuren vorliegen.

2. Dispersionen gemäss Anspruch 1, in welchen Diisocyanatotoluol-sulfonsäure-uretdion die disperse und Diisocyanatotoluol und/oder Diisocyanatodiphenylmethan die kontinuierliche Phase bilden.

3. Dispersionen gemäss Anspruch 1, in welchen Diisocyanatodiphenylmethan-sulfonsäureuretdion die disperse und Diisocyanatodiphenylmethan die kontinuierliche Phase bilden.

4. Verfahren zur Herstellung von Dispersionen gemäss Anspruch 1 durch Sulfonierung von aromatischen Polyisocyanaten, dadurch gekennzeichnet, dass man entweder

a) aromatische Polyisocyanate mit 0,05 bis 0,6 Mol Schwefeltrioxid pro Mol Polyisocyanat oder einer entsprechenden Menge eines Schwefeltrioxid enthaltenden oder bildenden Sulfonierungsmittels in Gegenwart von 0,2 bis 25 Gew.-%, bezogen auf das Gewicht des Polyisocyanats einer hydrophoben, keine gegenüber Isocyanatgruppen inerte hydrophile Substituenten aufweisenden, in Isocyanaten zumindest bei erhöhter Temperatur löslichen, bei Raumtemperatur festen oder flüssigen, polare Gruppen aufweisenden gegebenenfalls gegenüber Isocyanatgruppen reaktionsfähigen Gruppen aufweisenden, organischen Substanz sulfoniert oder

b) aromatische Polyisocyanate in Anwesenheit von 0,2 bis 25 Gew.-% bezogen auf das Gewicht des Polyisocyanats einer organischen Substanz der unter a) genannten Art in inerten, niedrig siedenden organischen Lösungsmitteln mit 0,2 bis 2,0 Mol Schwefeltrioxid pro Mol Polyisocyanat oder einer entsprechenden Menge eines Schwefeltrioxid enthaltenden oder bildenden Sulfonierungsmittels sulfoniert, anschliessend das Lösungsmittel destillativ entfernt, wobei bei Verwendung von mehr als 60 Mol-% Schwefeltrioxid pro Mol Polyisocyanat vor der destillativen Entfernung des Lösungsmittels das den wesentlichen Teil der kontinuierlichen Phase bildende Polyisocyanat zugesetzt wird, oder

c) instabile Grobsuspensionen von aromatischen Isocyanatosulfonsäure-uretdionen in Polyisocyanaten oder inerten Lösungsmitteln in Gegenwart von 0,2 bis 25 Gew.-%, bezogen auf die Gesamtisocyanatmenge an unter a) definierten organischen Substanzen kurzzeitig bis zum Entstehen einer Lösung auf maximal 150°C erhitzt und rasch abkühlt und/oder einer Mahlung unterwirft, wobei gegebenenfalls mitverwendetes organisches Lösungsmittel destillativ entfernt wird und im Falle der Verwendung von Grobsuspensionen, welche keine nicht-sulfonierten Polyisocyanate enthalten das die kontinuierliche Phase bildende organische Polyisocyanat vor der destillativen Entfernung des Lösungsmittels hinzugegeben wird, und wobei jeweils die Menge an aromatischem Isocyanatosulfonsäure-uretdion und nicht sulfoniertem Polyisocyanat so bemessen wird, dass in den letztlich erhaltenen stabilen Dispersionen, bezogen auf die Gesamtmenge der Äquivalente an Isocyanatgruppen, insgesamt 5 bis 60 Äquivalente-% an freien und dimerisierten Isocyanatgruppen des Isocyanatosulfonsäureuretdions vorliegen.

**Revendications**

1. Dispersions en fines particules, stables à la sédimentation, liquides à température ambiante ou liquéfiables par chauffage à une température maximale de 60°C, d'uret-diones d'acides isocyanatosulfoniques aromatiques solides en fines particules, à une dimension moyenne de particules inférieure à 0,02 mm, dans des polyisocyanates organiques, contenant, par rapport au nombre total des équivalents de groupes isocyanate, 5 à 60 équivalents % au total de groupes isocyanate libres et dimérisés d'acides polyisocyanatosulfoniques aromatiques.

2. Dispersions selon la revendication 1, dans lesquelles l'uret-dione d'acide diisocyanatotoluène et/ou le diisocyanatodiphénylméthane la phase continue.

3. Dispersions selon la revendication 1, dans lesquelles l'uret-dione d'acide diisocyanatodiphénylméthane-sulfonique forme la phase dispersée et le diisocyanatodiphényl-méthane la phase continue.

4. Procédé de préparation de dispersions selon la revendication 1, par sulfonation de polyisocyanates aromatiques, caractérisé en ce que:

a) ou bien on sulfone des polyisocyanates aromatiques par 0,05 à 0,6 mole, par mole de polyisocyanate, d'anhydride sulfurique ou une quantité correspondante d'un agent sulfonant contenant ou formant de l'anhydride sulfurique, en présence de 0,2 à 25% en poids, par rapport au poids du polyisocyanate, d'une substance organique hydrophobe ne portant pas de substituants hydrophiles inertes à l'égard des groupes isocyanate, soluble au moins à chaud dans les isocyanates, solide ou liquide à température ambiante, portant des groupes polaires, portant éventuellement des groupes réactifs à l'égard des groupes isocyanate;

b) ou bien on sulfone des polyisocyanates aromatiques en présence de 0,2 à 25% en poids, par rapport au poids du polyisocyanate, d'une substance organique du type mentionné en a) dans des solvants organiques inertes à bas point d'ébullition, par 0,2 à2,0 moles d'anhydride sulfurique par mole de polyisocyanate, ou par une quantité correspondante d'un agent sulfonant contenant ou formant de l'anhydride sulfurique, on élimine ensuite le solvant par distillation, étant précisé que lorsqu'on utilise plus de 60 moles % d'anhydride sulfurique par mole de polyisocyanate, on ajoute le polyisocyanate qui constitue la partie essentielle de la phase continue avant d'éliminer le solvant par distillation.

c) ou bien on chauffe un court moment à une température maximale de 150°C, jusqu'à formation d'une solution et on refroidit rapidement et/ou on soumet à un broyage des suspensions grossières instables d'uret-diones d'acides isocyanatosulfoniques aromatiques dans des polyisocyanates ou des solvants inertes en présence de 0,2 à 25% en poids, par rapport à la quantité totale d'isocyanate, de substance organiques du type défini en a) ci-dessus, étant précisé que le solvant organique éventuellement utilisé conjointement est éliminé par distillation et que dans le cas où on a utilisé des suspensions grossières ne contenant pas de polyisocyanates non sulfonés, on ajoute le polyisocyanate organique formant la phase continue avant d'éliminer le solvant par distillation, la quantité d'uret-dione d'acide isocyanatosulfonique aromatique et de polyisocyanate non sulfoné étant réglée dans chaque cas de manière que dans les dispersions stables obtenues finalement, il y ait au total de 5 à 60 équivalents % de groupes isocyanate libres et dimérisés de l'uret-dione d'acide isocyanatosulfonique par rapport à la quantité totale des équivalents de groupes isocyanate.

**Claims**

1. Finely divided, non-sedimenting dispersions in organic polyisocyanates of finely divided solid aromatic isocyanatosulphonic acid uret-diones

having a mean particle size of less than 0.02 mm which are liquid at room temperature or may be liquefied by heating to at most 60°C and which contain a total of from 5 to 60 equivalent % of free and dimerised isocyanate groups of aromatic polyisocyanatosulphonic acids, based on the total number of equivalents of isocyanate groups.

2. Dispersions according to claim 1 in which diisocyanatotoluene sulphonic acid uret-dione forms the disperse phase and diisocyanatotoluene and/or diisocyanatodiphenyl methane the continuous phase.

3. Dispersions according to claim 1 in which diisocyanatodiphenyl methane sulphonic acid uretdione forms the disperse phase and diisocyanatodiphenyl methane the continuous phase.

4. A process for producing the dispersions according to claim 1 by sulphonating aromatic polyisocyanates, characterised in that either:

a) aromatic polyisocyanates are sulphonated with from 0.5 to 0.6 moles of sulphur trioxide per mole of polyisocyanate or with a corresponding quantity of a sulphonating agent containing or forming sulphur trioxide in the presence of from 0.2 to 25% by weight, based on the weight of the polyisocyanate, of a hydrophobic organic substance which does not contain any hydrophilic substituents inert to isocyanate groups, is soluble in isocyanates, at least at an elevated temperature, is solid or liquid at room temperature and contains polar groups and, optionally, isocyanate-reactive groups, or

b) aromatic polyisocyanates are sulphonated with from 0.2 to 2.0 moles of sulphur trioxide per mole of polyisocyanate or with a corresponding quantity of a sulphonating agent containing or forming sulphur trioxide in the presence of from 0.2 to 25% by weight, based on the weight of the polyisocyanate, of an organic substance of the type mentioned in a) in inert low-boiling organic solvents, after which the solvent is removed by distillation, the polyisocyanate forming the major part of the continuous phase being added before removal of the solvent by distillation in cases where more than 60 mole % of sulphur trioxide is used per mole of polyisocyanate, or

c) unstable coarse suspensions of aromatic isocyanatosulphonic acid uret-diones in polyisocyanates or inert solvents are briefly heated to at most 150°C in the presence of from 0.2 to 25% by weight, based on the total quantity of isocyanate, of organic substances of the type defined in a) until a solution is formed, followed by rapid cooling and/or grinding, the organic solvent used, if any, being removed by distillation and, in cases where coarse suspensions which do not contain any non-sulphonated polyisocyanates are used, the organic polyisocyanate forming the continuous phase being added before removal of the solvent by distillation, and the quantity in which the aromatic isocyanatosulphonic acid uret-dione and the non-sulphonated polyisocyanate are used being gauged in such a way that the stable dispersions ultimately obtained contain a total of from 5 to 60 equivalent % of free and dimerised isocyanate groups of the isocyanatosulphonic acid uret-dione, based on the total number of equivalents of isocyanate groups.